# EUROPEAN PATENT APPLICATION

(11) **EP 3 865 033 A1**
(43) Date of publication of application: **18.08.2021**
(21) Application number: 19846431.5
(22) Date of filing: 08.08.2019
(51) Int. Cl.: A47K 3/28, A47K 7/02

(54) **SMART SPA SHOWER COLUMN**

(30) Priority: 08.08.2018 ES 201831254 U
(71) Applicant: INSETEC SURESTE S.L., 30006 Murcia (ES)
(72) Inventor: HERNÁNDEZ VICENTE, Juan José, 30100 Murcia (ES); HERNÁNDEZ VICENTE, Carlos, 30100 Murcia (ES); HERNÁNDEZ VICENTE, Raúl, 30100 Murcia (ES)
(74) Representative: Díaz Pacheco, Maria Desamparados
(86) International application number: PCT/ES2019/070558
(87) International publication number: WO 2020/030840

(57) **Abstract**

Smart spa shower column, comprising a compartment (8) containing a roller (2) which cleans it by pressure by means of a perforated tube (9) and which is located in the lower part of a chassis (1) that houses a motor and a spindle that provides vertical movement to a roller (2) that is attached to the chassis by means of arms (2a, 2b), one of which (2a) houses a motor which gives rotation to the roller (2) and the other arm (2b) houses a mechanism for locking the roller (2) in its stop position and a tube that provides water and/or soap to the roller; and wherein the chassis (1) houses an electronic control unit and a power supply battery. The chassis (1) is solidarily attached at the top to a shower system (3) which incorporates a dispenser (3a) and a presence sensor (5).

## Description

### Object of the invention

The object of this report is a smart spa shower column capable not only of providing a pleasant and soothing shower, but also of providing relaxation thanks to the effect of chromotherapy, creating a harmonious environment of warm lights, awakening sensations with scents thanks to the effect of aromatherapy and providing a therapeutic and soothing massage by means of a concealed roller that the user can activate at will, providing him or her with a fully customisable showering pattern suited to their characteristics, which, together with the adaptation and integration of new technologies, makes it stand out from other products on the market.

### Background to the invention

A wide variety of hydromassage and/or shower columns are currently available on the market, which combine different elements to act on how the water is released and to allow the user to increase or decrease the flow (for example, by means of the well-known rain effect or similar) that he or she receives, and where the sensation produced by this combination comparable to a gentle massage on the body of the person taking the shower.

Various models of shower and/or hydromassage columns are known in the state of the art, such as, for example, the one described in utility model ES 1 047 709 which describes an improved shower column intended to be used both in shower cubicles and in bathtubs, as a replacement for the corresponding conventional taps.

This model is characterised by a rigid bar attached to the wall, which emerges from a body of taps, preferably thermostatic, which can be fitted with a water outlet spout, complemented by a shower and movable arms with ducts which, at their end, are fitted with an adjustable hydromassage nozzle, thus creating an assembly of reduced dimensions to allow it to be fully used without constituting any obstacle for the user.

In a similar vein, patent ES 2309479 T3 describes an apparatus for body treatments using hydromassage, especially for the localised application of showers and massages by means of directed jets of water, of the type that comprises a flattened base for the rest and support of the user, and an articulated covering body with devices to open and close it which, with the flattened base, defines a housing for the location of the user in its interior during the treatment, a support base on the floor and a support frame. In addition, in a housing defined by the flattened base in the hinged covering body of the appliance, there is a spraying device consisting of a plurality of spray mechanisms, arranged between at least two horizontal and parallel platens or with a certain degree of parallelism, endowed with alternative movements in opposite directions the one to the other, and activated by means of devices with speed and travel stroke regulation, and because each of the sprinkler mechanisms comprises an axial tubular rod with a water inlet at one end and an outlet hole at the other, as well as a pair of spherical protuberances with associated cylindrical elements fitted with flanges and annular elements defining a pair of opposing ball-and-socket joints, intended to be coupled to the platens, respectively.

The same applicant is also the holder of utility model ES 1 104 905, which describes a shower device with a lathering and massaging head consisting of a frame, preferably with a parallelepiped geometry, which will have an enclosure covering it, a threaded rod connected to a motor and supported by a swivel bracket, a horizontal arm, a guide with a guide carriage, a secondary motor, a swivel head, an electronic board, a battery for powering the device and brackets for the swivel head, characterised in that, inside the frame, the threaded rod is arranged vertically, and said threaded rod is connected to a horizontal arm by means of an assembly part, wherein the threaded rod is connected to a motor which provides it with rotation, wherein the horizontal arm is significantly longer than the width of the frame so that its ends protrude from the sides of said frame, wherein at both ends of the arm there are brackets for the swivel head, wherein the arm has a vertical guide inside the frame to which it is attached by means of a carriage fitted with bearings, wherein at least one of the brackets has a motor that provides rotation to the rotating head, with all the electrical parts of the device being powered by a battery.

None of the inventions described, or known in the state of the art, solve the problem of designing a shower column that can be adapted to the physical characteristics of the user, and which integrates, at the same time, electronic devices that allow the user to interact with the shower itself, defining those elements that he or she considers most appropriate for their treatment and/or shower, in situ.

### Description of the invention

The technical problem solved by this invention is that of obtaining a shower column with a portable spa effect and with no technical requirements for the installation thereof, being comparable to the installation of any standard shower column existing on the market, adaptable to the physical characteristics of the user at any given time, with such parameters being able to be modified by and for several users, or directly by the user him or herself in situ, thanks to the presence of electronic devices that allow it to be manipulated. For this purpose, the smart spa shower column, which essentially comprises a chassis that houses the electronic boards necessary for the control of all the elements, electronic tap, battery, motor, spindle, lifting carriage and the shafts needed for the operation of a roller that is attached to the aforementioned lifting carriage by means of arms or brackets that run along guides located on the sides of the aforementioned chassis, which determine the length of the total displacement of the roller; and wherein the roller is essentially composed of a roller of latex, rubber or similar material, covered by a suitable textile material, sponge, horsehair, etc. and filled with air; and wherein the chassis incorporates in its upper part a presence sensor that determines the height of the user as well as the control of their environment, carrying out the necessary pre-established operations (shutdown in the event of absence, etc.). It is solidarily attached to a shower system that incorporates a rain, waterfall or similar type of head, and which is essentially made up of an electronic tap, controllable by means of a tablet that incorporates an integrated computer application, responsible for operating the different components of the system.

The power supply for the system is provided by a suitable external charger, which is plugged into a socket in the bathroom and supplies the permitted safety voltage (up to 24v DC and up to 12v AC) and charges the battery system as well as the standard power supply.

Thanks to its design, and to the presence of the inflatable roller that rotates and oscillates vertically at the same time, it improves the sensations when taking a shower, as this roller provides a pleasant massage (therapeutic treatment) on the neck, back, lumbar area, buttocks, thighs and calves, turning the shower into a space and a moment of relaxation for the user.

The column recommended here will be designed for any type of user, regardless of their size (it is recommended for children over the age of twelve, approximately), which, added to the fact that it is so easy to use, will make it an easily consumable product, which will increase the possibilities of it being integrated both into the private sphere of homes and in spaces of a more public nature, such as, for example, hotels, gyms or the like.

The fact that it incorporates a tablet with a specific mobile application means that the user can choose the parameters they want at any time, to obtain a greater sensation of comfort and/or relaxation, specifying the specific needs of each user by simply adjusting the settings on the tablet. In addition to this, given that it is an application, voice recognition, commands and control, facial recognition and all the developments that can be incorporated in accordance with the state of the art can be added to it, which brings such benefits as greater safety and ease of use, above all for the elderly, people with reduced mobility, etc.

The column will incorporate a sensor that adapts the oscillation of the roller to the height of the user, thus favouring the correct operation of the roller and avoiding possible accidents due to entrapment. The sensor adapts the height so that the roller does not go higher than neck height to thereby improve the user experience. This also means that the device is suitable for people with disabilities or the elderly, without compromising their physical integrity.

Moreover, while they are using the column, the user can use the tablet to perform other functions (both inside and outside the bathroom), such as watching videos, surfing the Internet, talking on the phone, listening to music or using their favourite applications. Thanks to the versatility of the tablet and its application which has been specially designed to control the device, the user can prepare the bath remotely, via Bluetooth® or similar, and can choose (among others): the temperature of the water; the flow rate; the speed, the direction of rotation and stop of the roller; the colour of the ambient light; whether they want more or less soap, etc.

The preferred embodiment of this invention, the column presented here, will not spill a single drop of water until the temperature programmed by the user is reached, thereby saving a large amount of water volume, which will result in both the economic savings achieved and in the optimisation of the existing water resources.

The roller consists essentially of a hollow cylinder made of latex or any similar material with two circular caps at the ends and crossed by a shaft. The whole assembly is watertight, covered with sponge, a suitable textile material, etc., whatever the user may prefer. There is a valve on one side so that the user can adjust the air pressure to his or her comfort (hardness of the roller). It is inserted into the arms by means of open and pressurised anchorages so that it can be easily inserted and extracted and so that each user can have their own roller. This also provides safety, as in the event of an entrapment or blockage, the roller will be released when the low pressure of the brackets is exceeded.

Finally, it will be easy to transport and install the column that is being presented here, which means that no specialised technical operators or civil works will be required for its installation and/or start-up. This will mean that it will be more widely marketable and that it can be sold in all types of DIY shops or shopping centres, which will increase the likelihood of profitability and economic success.

### Brief description of the figures

The following is a very brief description of a series of drawings that help to better understand the invention and that relate expressly to an embodiment of the invention, which is presented as a non-limiting example thereof.

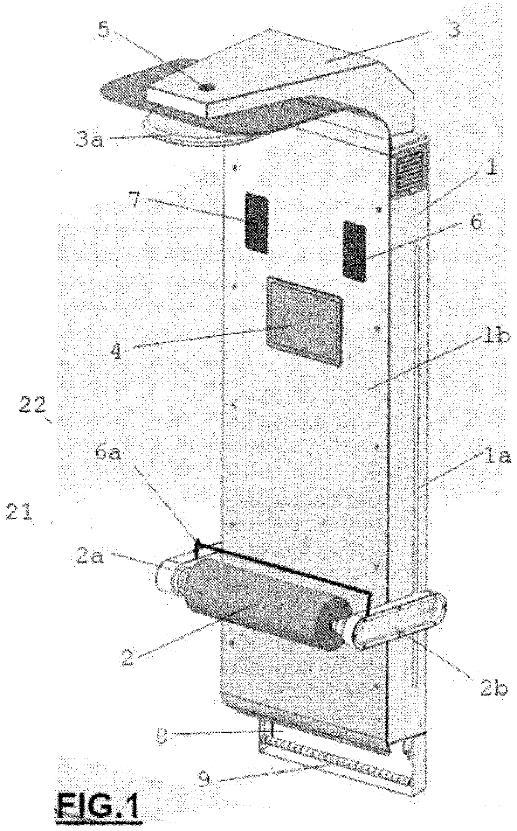

FIG 1. Shows a perspective view of the smart shower-spa column, which is the object covered by this utility model.

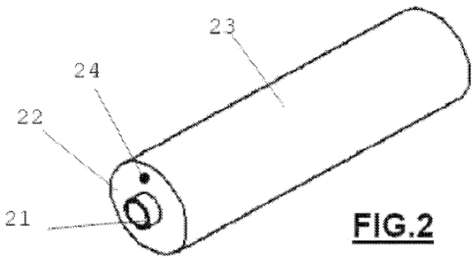

FIG 2. Shows a perspective view of the roller.

### Detailed illustration of an embodiment of the invention

A preferred embodiment of the invention is shown in the attached figures. More specifically, the smart spa shower column, which is the object covered by this report, is characterised in that it comprises a compartment (8) containing a roller (2) that is cleaned by pressure by means of a perforated tube (9) and which is located in the lower part of a chassis (1) that houses a motor, spindle and the elements needed to move a roller (2) which is attached to a lifting carriage (6a) by means of arms (2b) or brackets (2a) which run along guides (1a) located on the sides of said chassis (1), which will determine the length of the total displacement of the roller (2); in one of the arms (2a) a motor that provides the roller (2) with turning movement is housed; and wherein, in another arm (2b) a mechanism for blocking the roller (2) in its stop position is housed; and wherein, in said arm (2b) there is a hose or pipe that provides water and/or soap to the roller (2).

The chassis (1) will incorporate a front panel (1b) made of methacrylate or some other material with equivalent mechanical characteristics, which will protect the components housed therein and which will contain a series of recesses to house the elements that are accessible to the user, such as, for example, the tablet (4).

In a particular embodiment, the front panel (1b) will be a chromotherapy screen, operated by means of an application integrated into the tablet (4) or any other electronic device.

In a preferred embodiment, the roller (2), will preferably be composed of a hollow latex cylinder (23) covered by fabric and filled with air, in such a way that it adapts in the best possible way to the user's body, although other materials with equivalent mechanical characteristics are not ruled out. Similarly, the roller (2) is also composed of a shaft (21) that is coupled to the arms (2a, 2b) to transmit rotation, two rigid covers (22) that serve to support the assembly, and finally, a valve (24) that is responsible for pressurising the air that fills the roller (2). On the upper part of the chassis (1) there is a solidarily attached shower system (3) which incorporates a rain, waterfall or similar type of head (3a), and which is essentially composed of an electronic tap, controllable by means of a tablet (4) which incorporates an integrated computer application which controls the different components of the system, such as, for example, the movement of the trolley (6a), the water temperature, etc.

The column recommended here incorporates at least one presence sensor (5) that determines the height of the user to ensure that the roller (2) goes no further than the height of the user's neck. In a practical embodiment, the sensor (5) will be an ultrasonic sensor. In addition, in its lower part, the column incorporates a compartment (8) containing a roller (2) which cleans it by pressure by means of a perforated tube (9) after each use.

The tablet (4) is located on the front (1b) at a convenient height to allow it to be easily handled by the user and where it will be permanently charged for optimum operation. The tablet is removable so that it can be used as a remote control. Independently and as an alternative, the shower application can be downloaded from any of the currently existing repositories for installation on your own devices (smartphones, tablets, etc.) so that you can control the smart shower, without detriment to the individuality, ownership and security to which the user is entitled.

On the front panel (1b), at a convenient height, there is a soap tank (6) that feeds the roller (2) through a dispenser system, supplying it with water and soap as desired.

On the front panel (1b), at a convenient height, there is a tank for essential oils (7) or a similar product that will be used to provide aromatherapy.

In a preferred embodiment, the chassis (1) is made of stainless steel or another material with equivalent mechanical characteristics and will incorporate a series of anchorages that will allow it to be placed on any wall fitted for this purpose.

## Claims

1. Smart shower-spa column, **characterised in that** it includes a chassis (1) designed to house a motor, spindle and the elements required for the vertical movement of a roller (2) which is attached to a lifting carriage (1) by means of arms or brackets (2a) which run along guides (1a) located on the sides of said chassis (1), which will determine the length of the total displacement of the roller (2); and where, in one of the arms (2a) a motor that provides the roller (2) with turning movement is housed; and wherein, in another of the arms (2b) a mechanism for blocking the roller (2) in its stop position is housed; and where, in said arm (2b) there is a hose or pipe that is responsible for providing water and/or soap to the roller (2); and where, the chassis (1) houses the control electronics and power supply battery and the electronic tap that is controlled by the control unit; and where, the chassis (1), in its upper part, is solidarily attached to a shower system (3) that incorporates a rain, waterfall or similar type of head (3a) and a presence sensor (5) that determines the height of the user.

2. Smart spa shower column according to claim 1 wherein the presence sensor (5) is an ultrasonic sensor.

3. Smart shower-spa column according to claim 1 wherein the light screen (1b) incorporates a soap dispenser (6) which, by means of a dispensing system (6a), is responsible for supplying water and soap to the roller (2) as and when required by the user.

4. Smart spa shower column according to claim 1 wherein the front chassis (1) incorporates a front panel (1b) made of methacrylate or some other material with equivalent mechanical characteristics, which will protect the components housed therein, and which will have a series of recesses designed to house the elements that are accessible to the user, such as, for example, the tablet (4).

5. Smart shower-spa column according to claims 1 and 4 wherein the front panel (1b) is a chromotherapy screen, operated by means of an application integrated into the tablet (4) or any other electronic device.

6. Smart spa shower column according to claim 1 wherein the front chassis (1) made of stainless steel or another material with equivalent mechanical characteristics and will incorporate a series of anchorages that will allow it to be placed on any wall fitted for this purpose.

7. Smart shower-spa column according to claim 1 wherein the light screen (1b) incorporates a tank for essential oils (7), aromatherapy, which is controlled by the application, and which can be activated as and when required by the user.

8. Smart shower-spa column according to claim 1 which incorporates in its lower part a compartment (8) containing a roller (2) which cleans it by pressure by means of a perforated tube (9) after each use.

9. Smart spa shower column according to claim 1 wherein the roller (2) is composed of a shaft (21) which is coupled to the arms (2a, (2b) to transmit rotation, two rigid covers (22) that serve to support for the assembly, a hollow cylinder made of latex, rubber or similar material that seals the entire assembly and provides flexibility to ensure that the unit adapts correctly to the surface of the body; and finally, a valve (24) that is responsible for pressurising the air that fills the roller, and therefore, the hardness thereof, in accordance with the user's preferences.
